# EUROPÄISCHE PATENTSCHRIFT

(11) **EP 3 453 799 B1**
(45) Veröffentlichungstag und Bekanntmachung des Hinweises auf die Patenterteilung: **18.09.2019**
(21) Anmeldenummer: 18000711.4
(22) Anmeldetag: 03.09.2018
(51) Int. Cl.: E01C 19/28, E01C 19/35, E01C 19/38, E02D 3/026, E02D 3/046, G01N 33/24

(54) **VORRICHTUNG ZUR BODENVERDICHTUNG UND ÜBERWACHUNGSVERFAHREN**
SOIL COMPACTING DEVICE AND MONITORING METHOD
DISPOSITIF DE COMPACTAGE DE SOL ET PROCÉDÉ DE SURVEILLANCE

(30) Priorität: 11.09.2017 DE 102017008535
(43) Veröffentlichungstag der Anmeldung: 13.03.2019
(73) Patentinhaber: BOMAG GmbH, 56154 Boppard (DE)
(72) Erfinder: Laugwitz, Niels, 56154 Boppard (DE)
(74) Vertreter: Heidler, Philipp

(56) Entgegenhaltungen:
- WO-A1-2013/087783
- DE-B4-112010 000 670

## Beschreibung

Die Erfindung betrifft eine Vorrichtung zur Bodenverdichtung gemäß dem Oberbegriff des Patentanspruchs 1. Darüber hinaus betrifft die Erfindung ein Verfahren zum Überwachen von Veränderungen der mit einer Vorrichtung zur Bodenverdichtung erzeugten Verdichtung eines Bodens gemäß dem Oberbegriff des Patentanspruchs 11.

Gattungsgemäße Vorrichtungen zur Bodenverdichtung sind beispielsweise Anbauverdichter, die beispielsweise an einen Hydraulikarm eines Baggers als auswechselbares Werkzeug bzw. Anbaugerät angeschlossen werden können. Weitere gattungsgemäße Vorrichtungen zur Bodenverdichtung sind beispielsweise Verdichtungswalzen, wie Walzenzüge oder Tandemwalzen, die von einem Bediener von einem Fahrerstand aus gefahren werden können, sowie handgeführte Verdichtungswalzen, bei denen der Bediener neben der Verdichtungswalze läuft. Weitere gattungsgemäße handgeführte Vorrichtungen zur Bodenverdichtung umfassen Vibrationsstampfer, auch Rüttelstampfer genannt, und Vibrationsplatten, auch Rüttelplatten genannt. Gattungsgemäße Vorrichtungen zur Bodenverdichtung werden häufig im Straßen- und Wegebau, im Tiefbau sowie beim Anlegen von Plätzen etc. verwendet, um die Verdichtung des Bodens zu erhöhen und damit dessen Tragfähigkeit zu. verbessern. Die gattungsgemäßen Vorrichtungen weisen typischerweise einen Rahmen und einen vom Rahmen getragenen Antriebsmotor auf, der beispielsweise ein Verbrennungsmotor sein kann oder beispielsweise im Falle von Anbauverdichtern auch als Hydraulikmotor ausgebildet sein kann. Der Antriebsmotor treibt üblicherweise einen Schwingungserreger an, der mit einer Bodenverdichtungseinrichtung verbunden ist. Die Bodenverdichtungseinrichtung ist insbesondere eine Bodenplatte, typischerweise beispielsweise bei Anbauverdichtern, Vibrationsstampfern oder Vibrationsplatten, oder eine Walzenbandage, wie sie typischerweise bei selbstfahrenden und handgeführten Verdichtungswalzen vorgesehen ist. Um die Verdichtungsleistung der Vorrichtung zur Bodenverdichtung zu verbessern ist der Schwingungserreger typischerweise dazu ausgebildet, die Bodenverdichtungseinrichtung im Verdichtungsbetrieb bei einer festgelegten Vibrationsfrequenz oder innerhalb eines Vibrationsfrequenzbereiches in Schwingungen zu versetzen. Die Schwingungen werden von der Bodenverdichtungseinrichtung auf den zu verdichtenden Boden übertragen und unterstützen die Verdichtung des Bodenmaterials.

Es ist im Stand der Technik bekannt, auf der Vorrichtung zur Bodenverdichtung eine Umwandlungseinrichtung zur Umwandlung von Vibrationen in elektrische Energie vorzusehen, die Schwingungen der Vorrichtung zur Bodenverdichtung in elektrische Energie umwandelt, die zur Versorgung eines Verbrauchers eingesetzt wird. Häufig im Stand der Technik an Vorrichtungen zur Bodenverdichtung eingesetzte Verbraucher sind insbesondere wenigstens ein Sensor einer Sensoreinrichtung und/oder einer Sendeeinheit, die beispielsweise dazu ausgebildet sind, den Verdichtungsgrad des aktuell von der Vorrichtung zur Bodenverdichtung überfahrenen Bodens festzustellen und beispielsweise über eine Sendeeinheit an eine Einrichtung zur Auswertung der Daten zu übertragen. Der Verdichtungsgrad wird typischerweise als Bodensteifigkeit ermittelt, die mit zunehmender Verdichtung zunimmt. Aus dem Erreichen einer bestimmten Bodensteifigkeit kann also geschlossen werden, dass eine ausreichende Verdichtung des Bodens erreicht wurde. Eine Möglichkeit, die Bodensteifigkeit zu bestimmen, ist zusammen mit den mathematischen Grundlagen beispielsweise in der EP 2 627 826 D1 offenbart.

Eine weitere Vorrichtung zur Bodenverdichtung ist aus der DE112010000670B4 bekannt.

Der Vorteil darin, eine Umwandlungseinrichtung zur elektrischen Versorgung eines Verbrauchers einzusetzen, liegt darin, dass der Verbraucher nicht über fehleranfällige Kabelverbindungen von einer zusätzlichen Stromquelle, beispielsweise in einem Bordnetz der Vorrichtung zur Bodenverdichtung, mit Strom versorgt werden muss. Gerade wenn der Verbraucher an vibrierenden Bauteilen der Vorrichtung zur Bodenverdichtung angeordnet ist, sind zuverlässige Kabelverbindungen zur Versorgung des Verbrauchers kompliziert und teuer, weshalb es vorteilhaft ist, die notwendige elektrische Energie für den Verbraucher unabhängig von der restlichen Vorrichtung zur Bodenverdichtung durch die in der Nähe des Verbrauchers angeordnete Umwandlungseinrichtung zu gewinnen. Die Technik, durch eine Umwandlungseinrichtung elektrische Energie aus Vibrationen zu gewinnen, ist als *Energy Harvesting* bekannt. Diese Systeme basieren beispielsweise auf elektromagnetischer Induktion oder dem piezoelektrischen Effekt. Nachteilig ist, dass die Kombination von Sensoren zur Ermittlung des Verdichtungsgrades und Umwandlungseinrichtungen zur Versorgung des Sensors mit Strom relativ kostspielig sind.

Aufgabe der vorliegenden Erfindung ist es daher, die Komplexität einer Vorrichtung zur Bodenverdichtung in Bezug auf die Messung des Verdichtungsgrades des Untergrundes zu verringern und damit die Kosten zu senken.

Die Lösung dieser Aufgabe gelingt mit der Vorrichtung und den Verfahren gemäß den unabhängigen Ansprüchen. Bevorzugte Weiterbildungen sind in den abhängigen Ansprüchen angegeben.

Konkret gelingt die Lösung bei einer eingangs genannten gattungsgemäßen Vorrichtung zur Bodenverdichtung dadurch, dass die Umwandlungseinrichtung wenigstens zwei Feder-Masse-Systeme aufweist, die zueinander verschiedene Resonanzfrequenzen aufweisen, und dass eine Steuereinheit vorhanden ist, die aus der von den einzelnen Feder-Masse-Systemen gewonnenen elektrischen Energie, insbesondere der jeweiligen Spannung, den Verdichtungsgrad des Bodens ermittelt. Der Erfindung liegt dabei die Erkenntnis zugrunde, dass die tatsächliche Vibrationsfrequenz der Vorrichtung zur Bodenverdichtung vom aktuellen Verdichtungsgrad des Bodens beziehungsweise von der aktuellen Bodensteifigkeit abhängig ist. Vibriert die Vorrichtung zur Bodenverdichtung beispielsweise auf einem extrem weichen Boden, der der Vibrationsbewegung nur in vernachlässigbarem Maße entgegenwirkt, so schwingt die Vorrichtung zur Bodenverdichtung im Wesentlichen mit der durch den Schwingungserreger vorgegebenen Vibrationsfrequenz. Beim Verdichten eines Untergrundes treten allerdings auch Beschleunigungen mit zusätzlichen Frequenzkomponenten auf. Diese sind beispielsweise die doppelte Vibrationsfrequenz, die auch als Oberwelle bezeichnet wird, oder die halbe Vibrationsfrequenz, die auch als Unterwelle bezeichnet wird. Die Oberwelle beziehungsweise die Unterwelle bezeichnen also jeweils eine Schwingung der Vorrichtung zur Bodenverdichtung mit der doppelten beziehungsweise der halben durch den Schwingungserreger vorgegebenen Vibrationsfrequenz. Ein Feder-Masse-System bezeichnet dabei bekanntermaßen ein System, in dem ein gefedert gelagerter Körper mechanische Schwingungen ausführen kann. Vorliegend bezeichnet ein Feder-Masse-System dabei insbesondere den Schwingteil einer Umwandlungseinrichtung, die mechanische Schwingungen in elektrische Energie umwandelt. Durch die Verwendung von zwei Feder-Masse-Systemen mit unterschiedlichen Resonanzfrequenzen können die Resonanzfrequenzen so gewählt werden, dass die Umwandlungseinrichtung an jedem Feder-Masse-System frequenzselektiv/-abhängig elektrische Energie aus den Schwingungen der Vorrichtung zur Bodenverdichtung gewinnt. Dabei wird aus der voreingestellten Resonanzfrequenz des Feder-Masse-Systems und der entsprechenden Produktion von elektrischer Energie dieses Systems auf die Zusammensetzung der Frequenzkomponenten der Schwingung der Vorrichtung zur Bodenverdichtung rückgeschlossen.

Die Feder-Masse-Systeme werden durch die Vibration der Vorrichtung zur Bodenverdichtung in Schwingungen versetzt, die dann von der Umwandlungseinrichtung zur Gewinnung von elektrischer Energie genutzt werden. Die Umwandlung der Schwingungen der Feder-Masse-Systeme in elektrische Energie kann beispielsweise durch elektrische Induktion oder jede andere Methode des Energy Harvestings erfolgen. Die Schwingung des Feder-Masse-Systems erreicht dann ein Maximum, wenn die Vibration der Vorrichtung zur Bodenverdichtung eine Frequenz erreicht, die der Resonanzfrequenz des Feder-Masse-Systems entspricht. In diesem Bereich ist die Stromgewinnung durch die Umwandlungseinrichtung aufgrund der Schwingung dieses Feder-Masse-Systems besonders hoch. Durch die Anordnung von zwei Feder-Masse-Systemen mit zueinander verschiedener Resonanzfrequenz sind also zwei Frequenzbereiche verfügbar, in denen jeweils eines der Feder-Masse-Systeme mit der Resonanzfrequenz betrieben wird und daher die Umwandlungseinrichtung besonders viel elektrische Energie gewinnen kann, insbesondere an diesem Feder-Masse-System. Die Produktion der elektrischen Energie an den einzelnen Feder-Masse-Systemen wird dabei jeweils für sich separat voneinander gemessen.

Ist beispielsweise ein Feder-Masse-System auf die vom Schwingungserreger vorgegebene Vibrationsfrequenz eingestellt, sprich, die Resonanzfrequenz dieses Feder-Masse-Systems entspricht derjenigen Frequenz, mit der der Schwingungserreger die Vorrichtung zur Bodenverdichtung in Vibrationen versetzt, so produziert dieses Feder-Masse-System immer dann am meisten elektrische Energie, wenn die Vorrichtung zur Bodenverdichtung besonders weichen Boden verdichtet. Das zweite Feder-Masse-System kann beispielsweise auf die Oberwelle eingestellt sein. Das bedeutet, dass beispielsweise das zweite Feder-Masse-System eine Resonanzfrequenz aufweist, die der doppelten vom Schwingungserreger vorgegebenen Frequenz entspricht. Die Schwingung der Vorrichtung zur Bodenverdichtung mit der doppelten Vibrationsfrequenz des Schwingungserregers, sprich mit der Oberwelle, nimmt mit zunehmender Verdichtung des Bodens zu. Auf diese Weise produziert das zweite Feder-Masse-System proportional mehr elektrische Energie, wenn die Bodensteifigkeit beziehungsweise der Verdichtungsgrad des Untergrundes ansteigt. Ebenso wäre es möglich, das zweite Feder-Masse-System auf die halbe vom Schwingungserreger vorgegebene Schwingungsfrequenz einzustellen. In diesem Fall läge die Resonanzfrequenz des zweiten Feder-Masse-Systems also im Bereich der sogenannten Unterwelle. Schwingungen im Frequenzbereich der Unterwelle treten an der Vorrichtung zur Bodenverdichtung immer dann auf, wenn die Vorrichtung zur Bodenverdichtung auf dem Untergrund zu springen beginnt, sprich, wenn sie teilweise den Bodenkontakt verliert. Dies tritt insbesondere immer dann auf, wenn der Verdichtungsgrad des Untergrundes extrem hoch wird. Eine Zunahme der Produktion von elektrischer Energie des zweiten Feder-Masse-Systems wäre dann entsprechend ein Indikator für eine sehr hohe Bodensteifigkeit.

Der Kerngedanke der Erfindung liegt also darin, dass aus der jeweiligen Energieproduktion der Feder-Masse-Systeme der Umwandlungseinrichtung auf den Verdichtungsgrad des Untergrundes geschlossen werden kann. Dies kann beispielsweise anhand der Absolutwerte der Produktion von elektrischer Energie an den jeweiligen Feder-Masse-Systemen erfolgen. Alternativ ist es ebenso möglich, einen relativen Wert, beispielsweise aus dem Verhältnis der vom ersten und vom zweiten Feder-Masse-System produzierten elektrischen Energie zu bilden, beispielsweise durch Quotientenbildung oder Differenz der jeweils produzierten elektrischen Energie. Hierzu können Spitzenwerte und/oder Effektivwerte der elektrischen Energie beziehungsweise der elektrischen Kenngröße herangezogen werden. Auch aus diesem relativen Wert kann ein Rückschluss auf die Bodensteifigkeit beziehungsweise den Verdichtungsgrad des Bodens gezogen werden. Auf einen herkömmlichen Beschleunigungssensor, der zur Messung der Bodensteifigkeit eingesetzt wird, kann dann verzichtet werden. Insbesondere betrifft die Erfindung also eine Vorrichtung zur Bodenverdichtung, bei der der Verdichtungsgrad des Bodens ausschließlich aus der Produktion von elektrischer Energie an der Umwandlungseinrichtung ermittelt wird. Ein weiterer Vorteil der Erfindung liegt darin, dass die Umwandlungseinrichtung elektrischen Strom produziert und gleichzeitig keine von außen kommende Versorgungsspannung benötigen. Herkömmliche Beschleunigungssensoren erfordern eine Versorgungsspannung um ein Messsignal zu erzeugen. Auch herkömmliche Systeme, die Energy Harvesting einsetzen, nutzen eine separate Energy Harvesting-Einrichtung und betreiben mit der resultierenden elektrischen Energie einen Beschleunigungssensor. Das erfindungsgemäße System erzeugt elektrische Energie per Energy-Harvesting mit Hilfe mehrerer Feder-Masse-Systeme und macht somit eine separate Versorgung überflüssig, da die durch das Energy Harvesting produzierte elektrische Energie direkt und ohne einen weiteren Beschleunigungssensor zu versorgen zur Berechnung des Verdichtungsgrades des Bodens genutzt wird. Die Feder-Masse-Systeme sind dafür auf bestimmte Frequenzen abgestimmt, so dass beim Vergleich der erzeugten Energiemengen eine Aussage über die Verdichtung möglich wird. Die erfindungsgemäße Umwandlungseinrichtung ist daher unabhängig von einer externen Stromversorgung, sei es durch eine Batterie oder eine Verbindung zu einem elektrischen Netz auf der Vorrichtung zur Bodenverdichtung. Darüber hinaus weist die erfindungsgemäße Vorrichtung neben der Umwandlungseinrichtung keinen weiteren Beschleunigungssensor auf, insbesondere keinen Beschleunigungssensor, der mit elektrischer Energie versorgt werden muss, um zu funktionieren.

Die Resonanzfrequenzen der Feder-Masse-Systeme sind dabei vorteilhafterweise so gewählt, dass mindestens eines der Feder-Masse-Systeme beim Betrieb der Vorrichtung zur Bodenverdichtung, insbesondere beim Betrieb des Schwingungserregers, in Resonanz gerät. Um aus der gewonnenen elektrischen Energie auf den Verdichtungsgrad des Bodens zurück zu schließen, ist es weiter bevorzugt, dass die Resonanzfrequenzen der mindestens zwei Feder-Masse-Systeme in einem Verhältnis von 1:1,5 bis 1:3, bevorzugt von 1:1,5 bis 1:2,5, besonders bevorzugt 1:1,75 bis 1:2,25, ganz besonders bevorzugt von näherungsweise 1:2, zueinander stehen. Näherungsweise soll dabei bedeuten, dass das Verhältnis der Resonanzfrequenzen der mindestens zwei Feder-Masse-Systeme genau 1:2 beträgt, dabei allerdings kleine Abweichungen vernachlässigt werden, beispielsweise Herstellungs- oder Kalibrierungenauigkeiten.

Die Erfindung ist selbstverständlich nicht auf zwei Feder-Masse-Systeme beschränkt, sondern umfasst mindestens zwei Feder-Masse-Systeme. So können, um die Erfassung von Bodensteifigkeitsänderungen beziehungsweise Änderungen des Verdichtungsgrades des Bodens genauer zu erfassen, mehrere Feder-Masse-Systeme, beispielsweise drei, vier, fünf oder noch mehr Feder-Masse-Systeme, vorgesehen sein. Bevorzugt weisen sämtliche dieser Feder-Masse-Systeme zueinander verschiedene bzw. unterschiedliche Resonanzfrequenzen auf. Es kann allerdings auch vorgesehen sein, jeweils mehrere Feder-Masse-Systeme mit gleicher Resonanzfrequenz mit weiteren Feder-Masse-Systemen mit davon unterschiedlicher Resonanzfrequenz vorzusehen. Auf diese Weide wird die insgesamt produzierte elektrische Energie erhöht, da für jeden Resonanzfrequenzbereich mehrere schwingende Systeme bereitgestellt werden. Gleichzeitig werden die Signale, die einer bestimmten Bodensteifigkeit beziehungsweise einem bestimmten Verdichtungsgrad des Bodens zugeordnet sind, verstärkt. Beispielsweise könnten Paare von jeweils zwei, drei, vier oder fünf Feder-Masse-Systemen mit gleicher Resonanzfrequenz vorgesehen sein, die mit weiteren Paaren von Feder-Masse-Systemen mit unterschiedlicher Resonanzfrequenz kombiniert werden. Insgesamt ist durch die Kombination von beliebig vielen Feder-Masse-Systemen mit gleichen und/oder unterschiedlichen Resonanzfrequenzen eine Abdeckung eines beliebig großen Vibrationsfrequenzbereiches möglich, in dem die Umwandlungseinrichtung ausreichend elektrische Energie für mindestens einen Verbraucher zur Verfügung stellt und gleichzeitig den Verdichtungsgrad des Bodens misst. Erfindungsgemäß wichtig ist lediglich, dass immer zumindest zwei Feder-Masse-Systeme vorhanden sind, die zueinander verschiedene Resonanzfrequenzen aufweisen.

Wie bereits erläutert kann die Resonanzfrequenz der jeweiligen Feder-Masse-Systeme nach den typischen Vibrationsfrequenzen bzw. dem typischen Vibrationsfrequenzbereich gewählt werden, in dem die Vorrichtung zur Bodenverdichtung betrieben wird. Insbesondere liegen die Resonanzfrequenzen der Feder-Masse-Systeme innerhalb oder am Rand des während des Arbeitsbetriebes der Vorrichtung zur Bodenverdichtung typischerweise auftretenden Vibrationsfrequenzbereiches. Die gewählten Resonanzfrequenzen korrespondieren also mit dem bei der Bodenverdichtung mit der jeweiligen Vorrichtung typischerweise auftretenden Vibrationsfrequenzbereich. Dabei ist zu beachten, dass die konkrete Vibrationsfrequenz einer Vorrichtung zur Bodenverdichtung von einer Vielzahl Faktoren abhängt. Einerseits sind hier maschineneigene Parameter, wie beispielsweise die Betriebsbedingungen einer Erregereinrichtung, beispielsweise eines Unwuchterregers, maßgeblich. Andererseits wirken sich auch die äußeren Einsatzbedingungen auf die aktuelle Vibrationsfrequenz aus, wie beispielweise Eigenschaften des zu verdichtenden Bodenmaterials, beispielsweise dessen Steifigkeit. Wenn vorliegend somit von einer festgelegten Vibrationsfrequenz oder einem Vibrationsfrequenzbereich die Rede ist, bezieht sich dies insbesondere auf eine typische Einsatzsituation. Typische Vibrationsfrequenzen oder Vibrationsfrequenzbereiche können somit beispielweise empirisch ermittelt werden. Die Umwandlungseinrichtung ist also bevorzugt gezielt auf die Anwendung in einer spezifischen Vorrichtung zur Bodenverdichtung mit deren spezifischen Vibrationsfrequenzen ausgerichtet. Gibt es beispielsweise eine festgelegte Vibrationsfrequenz, in der die Vorrichtung zur Bodenverdichtung in einem Hauptteil des Verdichtungsbetriebs arbeitet, oder ist zumindest ein Mittelwert des Vibrationsfrequenzbereiches, der im Verdichtungsbetrieb der Vorrichtung zur Bodenverdichtung auftritt, bekannt, so kann beispielsweise eine der Resonanzfrequenzen der Feder-Masse-Systeme auf diesen Wert gelegt werden. In diesem Fall ist sichergestellt, dass in einem großen Teil des Verdichtungsbetriebes von diesem Feder-Masse-System eine ausreichende Schwingbewegung zur Verfügung steht, so dass die Umwandlungseinrichtung genügend elektrische Energie für mindestens einen Verbraucher produziert und gleichzeitig der Verdichtungsgrad des Bodens gemessen werden kann. Um allerdings schon mit nur zwei Feder-Masse-Systemen oder zumindest mit einer möglichst geringen Anzahl von Feder-Masse-Systemen einen möglichst großen Frequenzbereich abzudecken, ist es bevorzugt, dass die jeweilige Resonanzfrequenz der Feder-Masse-Systeme um eine festgelegte Vibrationsfrequenz oder den Mittelwert des Vibrationsbereiches herum festgelegt werden. So ist es beispielsweise bevorzugt, dass die zwei Feder-Masse-Systeme derart ausgebildet sind, dass die Resonanzfrequenz des einen Feder-Masse-Systems oberhalb der festgelegten Vibrationsfrequenz oder oberhalb des Mittelwertes des Vibrationsfrequenzbereiches und/oder die Resonanzfrequenz des anderen Feder-Masse-Systems unterhalb der Vibrationsfrequenz oder unterhalb des Mittelwertes des Vibrationsbereiches liegt. Je nachdem liegt also die Resonanzfrequenz des einen Feder-Masse-Systems näher an der Oberwelle oder der Unterwelle der Vibration, insbesondere im Vergleich zum jeweils anderen Feder-Masse-System. Durch die Zunahme der Produktion an elektrischer Energie dieses Feder-Masse-Systems wird also eine Erhöhung des Verdichtungsgrades des Bodens angezeigt. Gleichzeitig sollte die Umwandlungseinrichtung über den gesamten Vibrationsfrequenzbereich hinweg ausreichend elektrische Energie für mindestens einen Verbraucher zur Verfügung stellen. Je weiter die Resonanzfrequenzen der Feder-Masse-Systeme auseinander liegen, während das Kriterium der ausreichenden Energieproduktion über den gesamten Vibrationsfrequenzbereich hinweg weiterhin erfüllt bleibt, desto weiter ist der von den Feder-Masse-Systemen und damit der Umwandlungseinrichtung abgedeckte Vibrationsfrequenzbereich. Gleichzeitig sollten die Resonanzfrequenzen der Feder-Masse-Systeme allerdings ebenfalls möglichst nah an der Vibrationsfrequenz selbst, an der Oberwelle und/oder der Unterwelle liegen, um eine möglichst zuverlässige Messung des Verdichtungsgrads des Bodens und dessen Änderungen zu ermöglichen. Dass die Resonanzfrequenzen innerhalb des typischen Vibrationsfrequenzbereiches der Vorrichtung zur Bodenverdichtung liegen, ist ebenfalls ein Unterschied zu herkömmlichen Beschleunigungssensoren. Herkömmliche Beschleunigungssensoren sollen einen möglichst linearen Frequenzgang haben, also zumindest in einem gewissen Frequenzbereich (z.B. von 0 bis 400 Hz oder von 0 bis 20 kHz) immer die gleiche Empfindlichkeit (beispielsweise Volt pro m/s²) aufweisen. Dafür muss die stets vorhandene Resonanzfrequenz des Sensors erheblich über der maximalen Messfrequenz und damit dem typisch auftretenden Vibrationsfrequenzbereich liegen. Zudem wird bei herkömmlichen Beschleunigungssensoren oftmals die Dämpfung so weit erhöht, dass die Resonanzüberhöhung möglichst klein wird. So wird bei herkömmlichen Beschleunigungssensoren die Dämpfung des Sensorelementes häufig nahe des kritischen Dämpfungsgrads gewählt (Dämpfungsgrad D=1). Bei dem erfindungsgemäßen Feder-Masse-System wird nun bewusst die Resonanzfrequenz genau in den interessierenden Frequenzbereich gelegt, um sowohl genügend elektrische Energie zur Versorgung zu erzeugen als auch eine Bewertung des Frequenzinhalts der einwirkenden Schwingungen zu erhalten. Hierfür ist die Dämpfung eher klein zu halten, damit der Resonanzbereich schmal wird. Es ist daher bevorzugt, dass die Feder-Masse-Systeme eine geringe oder keine Dämpfung aufweisen. So kann der Dämpfungsgrad D der Feder-Masse-Systeme erfindungsgemäß beispielsweise <0,5, bevorzugt <0,4, weiter bevorzugt <0,3, weiter bevorzugt <0,2, weiter bevorzugt <0,1 sein. Die Feder-Masse-Systeme sind derart konstruiert, dass sie den entsprechenden Dämpfungsgrad D aufweisen, beispielsweise indem Reibungseffekte ausreichend stark vermieden werden.

In einer weiteren bevorzugten Ausführung der Erfindung sind die zwei Feder-Masse-Systeme derart ausgebildet, dass die Resonanzfrequenz des einen Feder-Masse-Systems bei der doppelten Vibrationsfrequenz oder beim doppelten Mittelwert des Vibrationsfrequenzbereiches (also im Bereich der Oberwelle) und/oder die Resonanzfrequenz des anderen Feder-Masse-Systems bei der halben Vibrationsfrequenz oder beim halben Mittelwert des Vibrationsfrequenzbereiches (also im Bereich der Unterwelle) liegt. Eine derartige Wahl der Resonanzfrequenzen der Feder-Masse-Systeme ist besonders geeignet, sowohl einen breiten Vibrationsfrequenzbereich abzudecken, als auch innerhalb dieses Bereiches bei jeder Vibrationsfrequenz ausreichende elektrische Energie durch die Umwandlungseinrichtung bereitstellen zu lassen. Darüber hinaus gelingt die Messung des Verdichtungsgrades des Untergrundes besonders gut, wenn die Resonanzfrequenzen der Feder-Masse-Systeme an der Oberwelle beziehungsweise der Unterwelle liegen. Besonders bevorzugt ist eine Ausführungsform, in der drei Feder-Masse-Systeme vorgesehen sind. Diese sind derart ausgebildet, dass die Resonanzfrequenz des ersten Feder-Masse-Systems bei der doppelten Vibrationsfrequenz oder beim doppelten Mittelwert des Vibrationsfrequenzbereiches und die Resonanzfrequenz des zweiten Feder-Masse-Systems bei der halben Vibrationsfrequenz oder beim halben Mittelwert des Vibrationsfrequenzbereiches und die Resonanzfrequenz des dritten Feder-Masse-Systems bei der Vibrationsfrequenz oder beim Mittelwert des Vibrationsfrequenzbereiches liegt. Mit anderen Worten liegen die Resonanzfrequenzen der drei Feder-Masse-Systeme also sowohl auf der Vibrationsfrequenz beziehungsweise auf dem Mittelwert des Vibrationsfrequenzbereiches, sowie einmal doppelt so hoch, also an der Oberwelle, und einmal halb so hoch, also an der Unterwelle. Auf diese Weise lässt sich der Verlauf der Veränderung der Bodensteifigkeit beziehungsweise des Verdichtungsgrads des Bodens während der Verdichtung besonders gut nachvollziehen. Zur Berechnung einer Verdichtungskennwertes kann hier beispielsweise ein Quotient aus den jeweiligen der Oberwelle und der Vibrationsfrequenz zugeordneten Messwerten herangezogen werden. Auch hier kommen Spitzenwerte und/oder Effektivwerte in Betracht. Es können auch Linearkombinationen aus den Quotienten Oberwelle/Vibrationsfrequenz und Unterwelle/Vibrationsfrequenz zur Berechnung herangezogen werden. Wie schon erläutert bezieht sich der Begriff "Vibrationsfrequenz" in diesem Zusammenhang auf die vom Schwingungserreger vorgegebene Grundfrequenz.

Die Umwandlungseinrichtung kann grundsätzlich an jeder Stelle der Vorrichtung zur Bodenverdichtung angeordnet werden, die im Verdichtungsbetrieb in Vibrationen versetzt wird. Typischerweise wird die Bodenverdichtungseinrichtung vom Schwingungserreger am stärksten in Vibrationen versetzt, teilweise sind andere Bereiche der Vorrichtung zur Bodenverdichtung gegenüber der Bodenverdichtungseinrichtung sogar schwingungsentkoppelt. Es ist daher bevorzugt, dass die Umwandlungseinrichtung an einem den Boden in direktem Bodenkontakt verdichtenden Bauteil angeordnet ist, insbesondere an einer Bodenplatte oder an einer Walzenbandage der Bodenverdichtungseinrichtung. Die Bodenverdichtungseinrichtung und insbesondere die Bodenplatte oder die Walzenbandage ist innerhalb der Vorrichtung zur Bodenverdichtung den größten Vibrationen ausgesetzt, wodurch auch am meisten Energie der Vibrationen auf die Umwandlungseinrichtung übertragen werden kann.

Die Umwandlungseinrichtung kann bevorzugt zusammen mit einem Verbraucher, insbesondere mit der Steuereinheit und/oder einer Sendeeinheit und/oder einer Anzeigeeinrichtung, Teil einer Baueinheit sein. Die Umwandlungseinrichtung und der Verbraucher bilden also ein Modul, das zusammen an einer beliebigen Stelle der Vorrichtung zur Bodenverdichtung montiert werden kann. Die Anbringung dieser Baueinheit ist besonders flexibel, da sie unabhängig von anderen Komponenten der Vorrichtung zur Bodenverdichtung arbeitet und lediglich darauf angewiesen ist, Vibrationen der Vorrichtung zur Bodenverdichtung aufnehmen zu können. Die Ausbildung als Baueinheit bedeutet dabei insbesondere, dass die Umwandlungseinrichtung und der Verbraucher gemeinsam in nur einem Montageschritt an der Vorrichtung zur Bodenverdichtung anbringbar sind. Bevorzugt ist die Umwandlungseinrichtung und der Verbraucher in der Baueinheit fest miteinander verbunden und als solche Baueinheit hergestellt worden. Eine derartige Baueinheit bzw. ein derartiges Model eignet sich auch als Nachrüstsatz für eine bereits bestehende Vorrichtung zur Bodenverdichtung.

Die Umwandlungseinrichtung kann an jeder Position der Bodenverdichtungseinrichtung angeordnet sein. Bevorzugt ist es, dass die Umwandlungseinrichtung derart an der Vorrichtung zur Bodenverdichtung angeordnet ist, dass sie entweder in Bezug auf eine Bodenkontaktfläche zentrisch oder in einem Randbereich liegt. So kann die Umwandlungseinrichtung beispielsweise in der Mitte der quer zu einer Arbeitsrichtung verlaufenden Bodenverdichtungseinrichtung oder in einem seitlichen Randbereich der Bodenverdichtungseinrichtung angeordnet sein.

Wie schon erwähnt kann die Umwandlungseinrichtung grundsätzlich jede geeignete Energy Harvesting-Technik umfassen. Beispielsweise umfasst die Umwandlungseinrichtung einen Permanentmagneten, insbesondere einen Permanentmagneten bestehend aus Neodym. Alternativ können auch mehrere Permanentmagneten vorgesehen sein. Ergänzend oder alternativ umfasst die Umwandlungseinrichtung wenigstens eine Schwingspule. Es kann also in der Umwandlungseinrichtung beispielsweise ein feststehender Magnet und eine bewegliche Spule oder auch eine feststehende Spule und ein beweglicher Magnet eingesetzt werden. Insbesondere ist es bevorzugt, dass die Umwandlungseinrichtung wenigstens einen Lineargenerator umfasst. Ein derartiger Generator, der eine Bewegung entlang einer geraden Linie in elektrische Energie umwandelt, ist besonders geeignet für die Anwendung zur Gewinnung von elektrischer Energie aus Vibrationsbewegungen und damit ebenfalls für die Ermittlung des Verdichtungsgrades des Untergrundes aus den entsprechenden Vibrationen in den jeweiligen Frequenzen.

Die erfindungsgemäße Umwandlungseinrichtung ist bevorzugt dazu ausgebildet, einen Verbraucher, insbesondere die Steuereinheit und/oder eine Sendeeinheit und/oder einer Anzeigeeinrichtung mit elektrischer Energie zu versorgen. Die Resonanzfrequenzen der Feder-Masse-Systeme sind insbesondere so gewählt, dass innerhalb eines Vibrationsfrequenzbereiches, der im Verdichtungsbetrieb der Vorrichtung zur Bodenverdichtung typischerweise vorkommt, immer zur Versorgung des wenigstens einen Verbrauchers ausreichende elektrische Energie von der Umwandlungseinrichtung bereitgestellt wird. Dabei nutzt die Umwandlungseinrichtung immer gleichzeitig sämtliche Feder-Masse-Systeme, wobei aufgrund der unterschiedlichen Resonanzfrequenzen der Feder-Masse-Systeme der Anteil an elektrischer Energie, der durch die Schwingung des jeweiligen Feder-Masse-Systems produziert wird, unterschiedlich groß ist. Während ein Feder-Masse-System im Resonanzfrequenzbereich betrieben wird, wird das andere Feder-Masse-System aufgrund der unterschiedlichen Resonanzfrequenz weniger stark in Schwingung versetzt und hat daher auch nur einen kleineren Anteil an der gesamten von der Umwandlungseinrichtung produzierten elektrischen Energie. Da der Vibrationsfrequenzbereich, in dem sich die Vibrationsfrequenzen im Verdichtungsbetrieb der Vorrichtung zur Bodenverdichtung normalerweise bewegen, bekannt ist, können die Resonanzfrequenzen der Feder-Masse-Systeme so gewählt werden, dass die Umwandlungseinrichtung immer ausreichend elektrische Energie zur Versorgung des mindestens einen Verbrauchers erzeugt. Auf diese Weise wird sichergestellt, dass über den gesamten Verdichtungsbetrieb hinweg die Versorgung des Verbrauchers mit elektrischer Energie gewährleistet wird. Insbesondere wird der wenigstens eine Verbraucher ausschließlich von der Umwandlungseinrichtung mit elektrischer Energie versorgt. Die Einheit aus Umwandlungseinrichtung und wenigstens einem Verbraucher ist also energetisch unabhängig von weiteren auf der Vorrichtung zur Bodenverdichtung eventuell noch vorhandenen Stromquellen, beispielsweise von einer Batterie oder einer sonstigen Stromquelle eines Bordnetzes, beispielsweise einer Lichtmaschine. Derartige Stromquellen können vorhanden sein, sind dann aber energetisch von der Umwandlungseinrichtung und insbesondere auch von dem wenigstens einen Verbraucher getrennt.

Wie eingangs bereits erwähnt, eignet sich die vorliegende Erfindung für sämtliche gattungsgemäßen Vorrichtungen zur Bodenverdichtung. Die erfindungsgemäße Vorrichtung zur Bodenverdichtung kann also insbesondere ein Anbauverdichter, ein Vibrationsstampfer bzw. ein Rüttelstampfer, eine Vibrationsplatte bzw. eine Rüttelplatte oder eine Vibrationswalze sein, wobei es keinen Unterschied macht, ob es eine handgeführte Vibrationswalze ist oder ob diese einen Fahrerstand für einen Bediener aufweist.

Die Lösung der eingangs genannten Aufgabe gelingt ebenfalls mit einem Verfahren zum Überwachen von Veränderungen der mit einer Vorrichtung zur Bodenverdichtung erzeugten Verdichtung eines Bodens, insbesondere einer Vorrichtung zur Bodenverdichtung, wie vorstehend beschrieben, umfassend die Schritte: Verdichten eines Bodens mit Hilfe einer über einen Schwingungserreger bei einer festgelegten Vibrationsfrequenz oder innerhalb eines Vibrationsfrequenzbereiches in Schwingungen versetzten Bodenverdichtungseinrichtung; Erzeugen von elektrischer Energie mit Hilfe einer Umwandlungseinrichtung zur Umwandlung von Vibrationen in elektrische Energie, wobei die Umwandlungseinrichtung wenigstens zwei Feder-Masse-Systeme aufweist, die zueinander verschiedene Resonanzfrequenzen aufweisen; Ermitteln und Überwachen der erzeugten elektrischen Energie oder eines damit korrespondierenden Parameters beider Feder-Masse-Systeme; und Korrelieren der ermittelten erzeugten elektrischen Energie oder eines damit korrespondierenden Parameters mit einer Veränderung der Verdichtung des Bodens, insbesondere der Bodensteifigkeit. Auch für das Verfahren gelten die Merkmale mit ihren Wirkungen und Vorteilen, die vorstehend zur erfindungsgemäßen Vorrichtung zur Bodenverdichtung beschrieben wurden, ebenfalls. Hier wird zur Vermeidung von Wiederholungen auf die vorstehenden Ausführungen Bezug genommen. Insbesondere ist es selbstverständlich auch im erfindungsgemäßen Verfahren möglich, mehr als zwei Feder-Masse-Systeme einzusetzen, solange wenigstens zwei dieser Feder-Masse-Systeme unterschiedliche Resonanzfrequenzen aufweisen. Das erfindungsgemäße Überwachungsverfahren bewirkt, dass ein separater Sensor für die Vibrationserfassung, beispielsweise ein Beschleunigungssensor, nicht notwendig ist. Dadurch werden die Komplexität und damit die Herstellungskosten der Vorrichtung zum Bodenverdichtung verringert.

Die Erfindung wird nachstehend anhand der in den Figuren gezeigten Ausführungsbeispiele näher erläutert. Es zeigen schematisch:
- Fig. 1A: eine Seitenansicht eines Anbauverdichters;
- Fig. 1B: eine Seitenansicht einer Verdichtungswalze;
- Fig. 1C: eine Seitenansicht eines Vibrationsstampfers;
- Fig. 1D: eine Seitenansicht einer Vibrationsplatte;
- Fig. 2: eine Baueinheit mit einer Umwandlungseinrichtung;
- Fig. 3: ein Frequenz-Amplituden-Diagramm der Schwingung der Feder-Masse-Systeme bei weichem Boden;
- Fig. 4: ein Frequenz-Amplituden-Diagramm der Schwingung der Feder-Masse-Systeme bei festerem Boden;
- Fig. 5: ein Frequenz-Amplituden-Diagramm der Schwingung der Feder-Masse-Systeme bei sehr festem Boden; und
- Fig. 6: ein Ablaufdiagramm des Verfahrens zum Überwachen von Veränderungen der mit einer Vorrichtung zur Bodenverdichtung erzeugten Verdichtung eines Bodens.

Gleiche bzw. gleich wirkende Bauteile sind in den Figuren mit den gleichen Bezugszeichen beziffert. Sich wiederholende Bauteile sind nicht in jeder Figur gesondert bezeichnet.

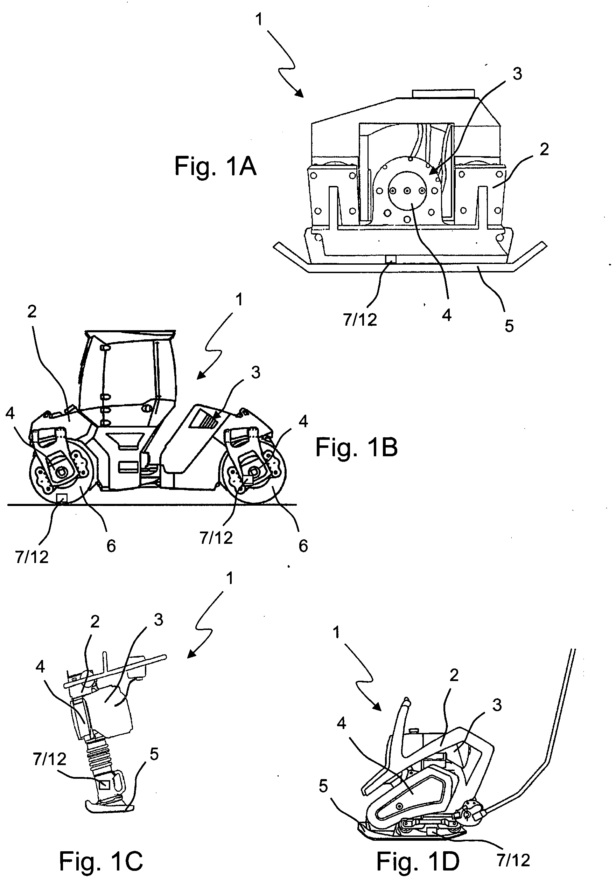

Die Figuren 1A, 1B, 1C und 1D zeigen jeweils gattungsgemäße Vorrichtungen 1 zur Bodenverdichtung. Konkret zeigt Fig. 1A einen Anbauverdichter, Fig. 1B eine Verdichtungswalze, Fig. 1C einen Vibrationsstampfer und Fig. 1D eine Vibrationsplatte. Die Vorrichtungen 1 zur Bodenverdichtung weisen alle einen Maschinenrahmen bzw. Rahmen 2 auf. Darüber hinaus umfassen sie einen Antriebsmotor 3, der beispielsweise bei der Verdichtungswalze der Fig. 1B, dem Vibrationsstampfer der Fig. 1C und der Vibrationsplatte der Fig. 1D ein Verbrennungsmotor, insbesondere einen Dieselverbrennungsmotor, ist. Im Fall des Anbauverdichters der Fig. 1A ist der Antriebsmotor 3 ein Hydraulikmotor, der beispielsweise mittels eines Schnellkupplungssystems mit dem Hydrauliksystem eines Baggers verbindbar und von diesem antreibbar ist. Der Antriebsmotor 3 treibt unter anderem einen Schwingungserreger 4 an, der die Bodenverdichtungseinrichtung der Vorrichtung 1 zur Bodenverdichtung in Schwingungen bzw. Vibrationen versetzt. Die Bodenverdichtungseinrichtung ist beim Anbauverdichter der Fig. 1A, beim Vibrationsstampfer der Fig. 1C und bei der Vibrationsplatte der Fig. 1D als Bodenplatte 5 und bei der Verdichtungswalze der Fig. 1B als Walzbandage 6 ausgebildet. Mit der Bodenplatte 5 bzw. der Walzbandage 6 wird die Vorrichtung 1 zur Bodenverdichtung im Verdichtungsbetrieb über den zu verdichtenden Boden geführt. Aufgrund des Eigengewichts der Vorrichtung 1 und der Vibrationsbewegung der Bodenverdichtungseinrichtung wird der von der Vorrichtung 1 überfahrene Boden zunehmend verdichtet. Zusätzlich weisen die Vorrichtungen 1 zur Bodenverdichtung eine Umwandlungseinrichtung 7 auf. Diese kann grundsätzlich überall an den Vorrichtungen 1 angeordnet sein, und ist beim Anbauverdichter der Fig. 1A und bei der Vibrationsplatte der Fig. 1D beispielsweise an der Bodenplatte 5 angeordnet. Beim Vibrationsstampfer der Fig. 1C ist die Umwandlungseinrichtung beispielhaft am Stampferfuß befestigt, könnte allerdings beispielsweise ebenso an anderen Stellen der Vorrichtung 1, beispielsweise ebenfalls an der Bodenplatte 5, angeordnet sein. Bei der Verdichtungswalze der Fig. 1B ist jeweils eine Umwandlungseinrichtung an jeder der beiden Walzbandagen 6 angeordnet, wobei die Umwandlungseinrichtung beispielhaft an der einen Walzbandage 6 am Innenumfang des Innenmantels der als Hohlzylinder ausgebildeten Walzbandage angeordnet ist, während sie sich bei der anderen Walzbandage 6 am Bandagenlager befindet. Wie schon beschrieben, sind diese Anordnungspositionen der Umwandlungseinrichtung 7 allerdings nur beispielhaft.

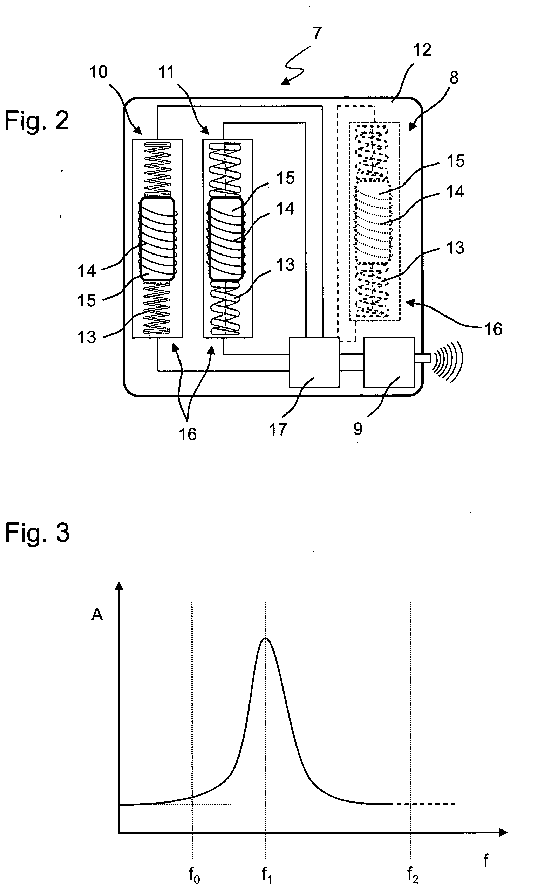

Fig. 2 zeigt den Aufbau einer Umwandlungseinrichtung 7, wie sie bei den Vorrichtungen 1 zur Bodenverdichtung der Figuren 1A bis 1D eingesetzt wird. Insgesamt weist die Umwandlungseinrichtung 7 zwei Lineargeneratoren 16 auf, die hier als erstes Feder-Masse-System 10 und zweites Feder-Masse-System 11 ausgebildet sind. Gestrichelt ist optional ebenfalls ein drittes Feder-Masse-System 8 angedeutet, das ebenfalls in der Umwandlungseinrichtung 7 angeordnet und von dieser umfasst sein kann. Jedes Feder-Masse-System 10, 11, 8 umfasst wenigstens eine, im gezeigten Ausführungsbeispiel sogar zwei Federn 13, die einen Permanentmagneten 15 unter Federvorspannung beweglich, insbesondere linear beweglich, lagern. Um den Permanentmagneten 15 herum ist eine Spule 14 aus elektrisch leitendem Material, beispielsweise Kupferdraht, angeordnet, so dass durch eine Bewegung des Permanentmagneten 15 innerhalb der Spule 14 ein elektrischer Strom in der Spule 14 induziert wird. Aufgrund der federnden Lagerung der Permanentmagnete 15 ist die Umwandlungseinrichtung 7 vibrationsempfindlich ausgebildet. Dies bedeutet, dass die Umwandlungseinrichtung 7, wenn sie in Vibrationen versetzt wird, in den Lineargeneratoren 16 elektrische Energie gewinnt. Mit der gewonnenen elektrischen Energie versorgt die Umwandlungseinrichtung 7 im gezeigten Ausführungsbeispiel eine Steuereinheit 17 und eine Sendeeinheit 9. Die Steuereinheit 17 misst beispielsweise die von den jeweiligen Feder-Masse-Systemen 10, 11, 8 produzierte elektrische Energie und gibt die gewonnenen Daten, entweder unverarbeitet oder verarbeitet, an die Sendeeinheit 9 weiter. Die Sendeeinheit 9 überträgt die Daten kabellos an eine Empfangseinrichtung (nicht dargestellt). Hierzu kommen sämtliche Möglichkeiten für eine Funkübertragung in Betracht, beispielsweise WLAN, Bluetooth, eine Infrarotschnittstelle und dergleichen. Die Umwandlungseinrichtung 7 ist zusammen mit der Steuereinrichtung 17 und der Sendeeinheit 9 als Baueinheit 12 ausgebildet. Die Baueinheit 12 ist ein einzelnes, vorgefertigtes Bauteil, das wie ein Modul insgesamt und zusammenhängend in nur einem Montageschritt an einer Vorrichtung 1 zur Bodenverdichtung montierbar ausgebildet ist. Die Baueinheit 12 kann auch an schon bestehende Vorrichtungen 1 zur Bodenverdichtung angebaut werden und eignet sich daher auch als Nachrüstsatz.

Die Fig. 3, 4 und 5 zeigen jeweils ein Frequenz-Amplituden-Diagramm, in dem die Frequenz f auf der Abszisse und die Amplitude A auf der Ordinate eingetragen ist. Die Frequenz f₁ ist die Vibrationsfrequenz beziehungsweise der Mittelwert des Vibrationsfrequenzbereiches, die beziehungsweise der vom Schwingungserreger 4 vorgegeben wird. Die Frequenz f₂ ist die doppelte Frequenz von f₁, während die Frequenz f₀ die halbe Frequenz von f₁ ist. Die Frequenz f₂ ist daher die Oberwelle und f₀ die Unterwelle der Vibrationsfrequenz f₁. Die in den Figuren 3-5 jeweils eingetragenen Graphen zeigen den Verlauf der Amplituden der Feder-Masse-Systeme 10, 11, 8 in Abhängigkeit der Frequenz f. Im gezeigten Ausführungsbeispiel betrifft der Graph mit der durchgezogenen Linie das erste Feder-Masse-System 10, dessen Resonanzfrequenz f₁ mit der vom Schwingungserreger 4 vorgegebenen Vibrationsfrequenz f₁ übereinstimmt, der gestrichelte Graph das zweite Feder-Masse-System 11, dessen Resonanzfrequenz f₂ doppelt so groß ist wie die vom Schwingungserreger 4 vorgegebenen Vibrationsfrequenz f₁ und der gepunktete Graph das dritte Feder-Masse-System 8, dessen Resonanzfrequenz f₀ halb so groß ist wie die vom Schwingungserreger 4 vorgegebene Vibrationsfrequenz f₁. Bei einer Anregung der Umwandlungseinrichtung 7 und damit der Feder-Masse-Systeme 10, 11, 8 bei den Resonanzfrequenzen f₀, f₁ oder f₂ kommt es für die jeweilige Resonanzfrequenz f₀, f₁ oder f₂ beim entsprechenden Feder-Masse-System 10, 11, 8 zu einem erheblichen Anstieg der Amplitude A. Bei einer größeren Amplitude A wird der Permanentmagnet 15 schneller und weiter durch die Spule 14 hindurchbewegt, wodurch in der Spule 14 mehr elektrische Energie erzeugt wird, beispielsweise eine höhere Spannung entsteht. Da die Verbraucher, beispielsweise die Steuereinheit 17 und die Sendeeinheit 9 oder eine Anzeigeeinrichtung (nicht dargestellt), immer mit ausreichender elektrischer Energie versorgt werden müssen, ist es wichtig, dass die von allen Lineargeneratoren 16 bereitgestellte elektrische Energie zusammen zur Versorgung der Verbraucher ausreicht.

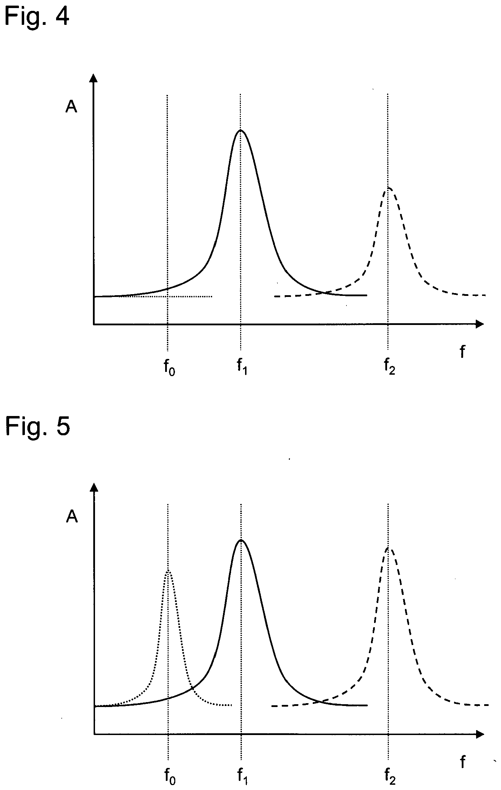

Figur 3 zeigt den Betrieb der Vorrichtung zur Bodenverdichtung auf sehr weichem Untergrund. Das Bild entspricht im Wesentlichen demjenigen, wenn die Bodenverdichtungseinrichtung in der Luft frei vibrieren würde. Die Schwingung der Bodenverdichtungseinrichtung entspricht im Wesentlichen einer Schwingung in der Vibrationsfrequenz f₁, also derjenigen Frequenz, die vom Schwingungserreger 4 vorgegeben wird. Da diese Frequenz f₁ ebenfalls der Resonanzfrequenz des ersten Feder-Masse-Systems 10 entspricht, schwingt das erste Feder-Masse-System 10 entsprechend mit einer hohen Amplitude, wie in der Figur dargestellt. Die beiden weiteren Feder-Masse-Systeme 11, 8 schwingen dagegen so gut wie nicht, was durch die horizontale gestrichelte beziehungsweise gepunktete Linie bei den jeweiligen Resonanzfrequenzen f₂ und f₀ angedeutet ist. Figur 4 zeigt den Betrieb der Vorrichtung zur Bodenverdichtung auf einem Untergrund, der schon deutlich fester ist als derjenige gemäß Figur 3. Dies kann beispielsweise daran liegen, dass der Boden bereits zu einem gewissen Grad verdichtet wurde. Aufgrund des festeren Untergrundes hat sich die Schwingungsantwort des Bodens verändert, wodurch die Schwingung der Bodenverdichtungseinrichtung nicht mehr nur aus der vom Schwingungserreger 4 vorgegebenen Vibrationsfrequenz f₁ besteht. Zusätzlich zur Vibrationsfrequenz f₁ umfasst die Schwingung der Bodenverdichtungseinrichtung in diesem Fall ebenfalls eine Komponente mit der doppelten Frequenz f₂, der sogenannten Oberwelle. Da die Umwandlungseinrichtung 7 ein zweites Feder-Masse-System 11 umfasst, dessen Resonanzfrequenz f₂ auf die Oberwelle eingestellt ist, beginnt also nun in diesem Stadium ebenfalls das zweite Feder-Masse-System 11 in nennenswerter Weise zu schwingen und dabei elektrische Energie zu produzieren. Bei einer weiteren Erhöhung der Bodensteifigkeit beziehungsweise bei einer weiteren Erhöhung des Verdichtungsgrades des Untergrundes ergibt sich dann schließlich die in Figur 5 dargestellte Situation. Figur 5 zeigt ein Betriebsstadium, in dem der Untergrund bereits so stark verdichtet ist, dass die Bodenverdichtungseinrichtung auf diesem zu springen beginnt und teilweise den Bodenkontakt verliert. Aus diesem Grund entsteht eine neue Schwingungskomponente in der Schwingung der Bodenverdichtungseinrichtung mit der Frequenz f₀, die halb so groß ist wie die vom Schwingungserreger 4 vorgegebene Vibrationsfrequenz f₁. Aus diesem Grund beginnt ebenfalls das dritte Feder-Masse-System 8, dessen Resonanzfrequenz auf die Unterwelle mit der Frequenz f₀ eingestellt ist, in nennenswerter Weise zu schwingen und elektrische Energie zu produzieren. Die jeweils an den einzelnen Feder-Masse-Systemen 10, 11, 8 produzierte elektrische Energie wird von der Steuereinheit 17 registriert. Wie insbesondere aus einer Zusammenschau der Figuren 3, 4 und 5 hervorgeht, ist erkennbar, dass bei ansteigender Bodensteifigkeit die einzelnen Feder-Masse-Systeme 10, 11, 8 unterschiedliche Signale, insbesondere in Relation zueinander, liefern, die einen Rückschluss auf die Bodensteifigkeit beziehungsweise auf den Verdichtungsgrad des Bodens zulassen. Es kann nun beispielsweise vorgesehen sein, dass die Steuereinheit 17 bereits die von den Feder-Masse-Systemen 10, 11, 8 aufgenommenen Signale in eine Angabe der Bodensteifigkeit umrechnet. Alternativ kann es ebenfalls vorgesehen sein, dass die Steuereinheit 17 die Messwerte unverarbeitet weitergibt und die Ermittlung der Bodensteifigkeit beziehungsweise des Verdichtungsgrades später erfolgt. Die Daten werden beispielsweise an die Sendeeinheit 9 weitergegeben, die diese kabellos an eine Empfangseinrichtung übermittelt. Die Empfangseinrichtung sorgt dann beispielsweise für eine Weiterverarbeitung der Daten und/oder eine Anzeige der Bodensteifigkeit für den Bediener der Vorrichtung zur Bodenverdichtung. Die Anzeige kann allerdings auch direkt an der Baueinheit 12 vorgesehen sein, beispielsweise über ein hier angebrachtes Display, das von außen für einen Bediener sichtbar ist. Die Ermittlung der Bodensteifigkeit erfolgt dabei in der Art, dass ein Anstieg der Produktion von elektrischer Energie des Feder-Masse-Systems 11, dessen Resonanzfrequenz f₂ der doppelten Vibrationsfrequenz f₁ entspricht, insbesondere im Vergleich zur Stromproduktion des Feder-Masse-Systems 10, dessen Resonanzfrequenz der Vibrationsfrequenz f₁, die durch den Schwingungserreger 4 vorgegeben ist, entspricht, in einen entsprechenden Anstieg der Bodensteifigkeit umgerechnet wird. Ein Anstieg der Produktion von elektrischer Energie des Feder-Masse-Systems 8, dessen Resonanzfrequenz f₀ auf die halbe Vibrationsfrequenz f₁ eingestellt ist, wird ebenfalls in einen noch größeren Anstieg der Bodensteifigkeit umgerechnet. Auf diese Weise kann die Bodensteifigkeit allein anhand der Produktion von elektrischer Energie der einzelnen Feder-Masse-Systeme 10, 11, 8 ermittelt werden, wodurch auf einen herkömmlichen Beschleunigungssensor zur Messung der Bodensteifigkeit verzichtet werden kann.

Die von verschiedenen Vorrichtungen 1 zur Bodenverdichtung beziehungsweise deren Schwingungserregern 4 vorgegebenen Vibrationsfrequenzen variieren je nach Art der Vorrichtung 1 zur Bodenverdichtung und ebenfalls je nach Modell und Ausführung. So liegen die Vibrationsfrequenzen von Anbauverdichtern üblicherweise im Bereich von 35 bis 60 Hz, von Rüttelplatten im Bereich von 35 bis 100 Hz, von Walzen im Bereich von 25 bis 75 Hz und von Stampfern im Bereich von 10 bis 14 Hz. Bei einem Anbauverdichter mit beispielsweise 45 Hz Arbeitsfrequenz müsste also ein Feder-Masse-System auf f₁ = 45 Hz abgestimmt sein. Ein weiteres Feder-Masse-System dann entsprechend auf f₂ = 90 Hz, also die doppelte Arbeitsfrequenz. Ein weiteres Feder-Masse-System könnte dann ebenfalls beispielsweise auf die Unterwelle bei f₀ = 22,5 Hz ausgerichtet sein. Zur Ermittlung könnte dann das Verhältnis der Oberwellenamplitude (90 Hz) zur Grundschwingungsamplitude (45 Hz) ermittelt werden. Dieses Verhältnis steigt mit zunehmender Verdichtung. Es ist ebenfalls möglich, dass der Anbauverdichter zwei Arbeitsfrequenzen, beispielsweise 45 Hz und 60 Hz, aufweist. In diesem Fall könnte als Kompromiss die Resonanzfrequenz eines Feder-Masse-Systems auf f₁ = 52,5 Hz gesetzt werden und die Resonanzfrequenz eines weiteren Feder-Masse-Systems auf f₂ = 105 Hz. Mit dieser Konfiguration wäre eine ausreichende Anregung der Feder-Masse-Systeme sowohl bei 45 Hz als auch bei 60 Hz Arbeitsfrequenz gegeben, so dass ein nutzbares Signal generiert werden kann. Die angegebenen Beispiele zum Anbauverdichter sind auf die weiteren Vorrichtungen 1 zu Bodenverdichtung übertragbar, in entsprechender Anwendung auf die üblichen Vibrationsfrequenzen dieser Vorrichtungen 1. Dabei ist darauf zu achten, dass die Resonanzfrequenzen der Feder-Masse-Systeme derart gewählt werden, dass bei den entsprechenden Betriebsfrequenzen eine ausreichend hohe Verstärkung von mindestens 2 gegeben ist. Diese Mindestanforderung gilt für sämtliche Ausführungen der vorliegenden Erfindung.

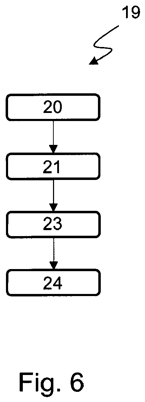

Fig. 6 zeigt ein Ablaufdiagramm des erfindungsgemäßen Verfahrens 19 zum Überwachen von Veränderungen der mit einer Vorrichtung 1 zur Bodenverdichtung erzeugten Verdichtung eines Bodens. Das Verfahren 19 beginnt mit dem Verdichten 20 eines Bodens mit Hilfe einer über einen Schwingungserreger 4 in Schwingungen versetzten Bodenverdichtungseinrichtung. Währenddessen erfolgt das Erzeugen 21 von elektrischer Energie mit Hilfe der Umwandlungseinrichtung 7, wie vorstehend erläutert. Insbesondere wird hierbei auf wenigstens zwei Feder-Masse-Systeme 10, 11, 8 zurückgegriffen, die zueinander verschiedene Resonanzfrequenzen f₀, f₁, f₂ aufweisen. Im Verfahren 19 erfolgt weiterhin ein Ermitteln und Überwachen 23 der erzeugten elektrischen Energie oder eines damit korrespondierenden Parameters jedes einzelnen Feder-Masse-Systems 10, 11, 8 und ein Korrelieren 24 der ermittelten erzeugten elektrischen Energie oder des damit korrespondierenden Parameters mit der Veränderung der Verdichtung des Bodens, ebenfalls wie vorstehend beschrieben. Auf diese Weise lässt sich das Vorsehen eines separaten Beschleunigungssensor zur Ermittlung der Bodensteifigkeit einsparen, wodurch die Vorrichtung 1 zur Bodenverdichtung insgesamt kostengünstiger ist.

## Patentansprüche

1. Vorrichtung (1) zur Bodenverdichtung, umfassend
a) einen Rahmen (2) und einen vom Rahmen (2) getragenen Antriebsmotor (3),
b) einen vom Antriebsmotor (3) angetriebenen Schwingungserreger (4), und
c) eine Bodenverdichtungseinrichtung, insbesondere eine Bodenplatte (5) oder eine Walzenbandage (6), die mit dem Schwingungserreger (4) verbunden ist,
wobei der Schwingungserreger (4) die Bodenverdichtungseinrichtung im Verdichtungsbetrieb bei einer festgelegten Vibrationsfrequenz oder innerhalb eines Vibrationsfrequenzbereiches in Schwingungen versetzt, und wobei eine Umwandlungseinrichtung (7) zur Umwandlung von Vibrationen in elektrische Energie vorhanden ist, die Schwingungen der Vorrichtung (1) zur Bodenverdichtung in elektrische Energie umwandelt,
**dadurch gekennzeichnet,**
**dass** die Umwandlungseinrichtung (7) mindestens zwei Feder-Masse-Systeme (10, 11) aufweist, die zueinander verschiedene Resonanzfrequenzen aufweisen, und dass eine Steuereinheit (17) vorhanden ist, die aus der von den einzelnen Feder-Masse-Systemen (10, 11) gewonnenen elektrischen Energie, insbesondere der jeweiligen Spannung, den Verdichtungsgrad des Bodens ermittelt.

2. Vorrichtung (1) zur Bodenverdichtung gemäß Anspruch 1,
**dadurch gekennzeichnet,**
**dass** die Resonanzfrequenzen der mindestens zwei Feder-Masse-Systeme (10, 11) in einem Verhältnis von 1:1,5 bis 1:3, bevorzugt von 1:1,5 bis 1:2,5, besonders bevorzugt 1:1,75 bis 1:2,25, ganz besonders bevorzugt von näherungsweise 1:2, zueinander stehen.

3. Vorrichtung (1) zur Bodenverdichtung gemäß einem der vorhergehenden Ansprüche,
**dadurch gekennzeichnet,**
**dass** die zwei Feder-Masse-Systeme (10, 11) derart ausgebildet sind, dass die Resonanzfrequenz des einen Feder-Masse-Systems (10) oberhalb der festgelegten Vibrationsfrequenz oder oberhalb des Mittelwertes des Vibrationsfrequenzbereiches und/oder die Resonanzfrequenz des anderen Feder-Masse-Systems (11) unterhalb der Vibrationsfrequenz oder unterhalb des Mittelwertes des Vibrationsfrequenzbereiches liegt.

4. Vorrichtung (1) zur Bodenverdichtung gemäß einem der vorhergehenden Ansprüche,
**dadurch gekennzeichnet,**
**dass** die zwei Feder-Masse-Systeme (10, 11) derart ausgebildet sind, dass die Resonanzfrequenz des einen Feder-Masse-Systems (10) bei der doppelten Vibrationsfrequenz oder beim doppelten Mittelwert des Vibrationsfrequenzbereiches und/oder die Resonanzfrequenz des anderen Feder-Masse-Systems (11) bei der halben Vibrationsfrequenz oder beim halben Mittelwert des Vibrationsfrequenzbereiches liegt.

5. Vorrichtung (1) zur Bodenverdichtung gemäß einem der vorhergehenden Ansprüche,
**dadurch gekennzeichnet,**
**dass** sie drei Feder-Masse-Systeme (10, 11, 8) umfasst, die derart ausgebildet sind, dass die Resonanzfrequenz f₁ des ersten Feder-Masse-Systems (10) bei der Vibrationsfrequenz oder beim Mittelwert des Vibrationsfrequenzbereiches und die Resonanzfrequenz f₂ des zweiten Feder-Masse-Systems (11) bei der doppelten Vibrationsfrequenz oder beim doppelten Mittelwert des Vibrationsfrequenzbereiches und die Resonanzfrequenz f₀ des dritten Feder-Masse-Systems (8) bei der halben Vibrationsfrequenz oder beim halben Mittelwert des Vibrationsfrequenzbereiches liegt.

6. Vorrichtung (1) zur Bodenverdichtung gemäß einem der vorhergehenden Ansprüche,
**dadurch gekennzeichnet,**
**dass** die Umwandlungseinrichtung (7) an einem den Boden im direkten Bodenkontakt verdichtenden Bauteil angeordnet ist, insbesondere an einer Bodenplatte (5) oder an einer Walzenbandage (6) der Bodenverdichtungseinrichtung.

7. Vorrichtung (1) zur Bodenverdichtung gemäß einem der vorhergehenden Ansprüche,
**dadurch gekennzeichnet,**
**dass** die Umwandlungseinrichtung (7) wenigstens eines der folgenden Merkmale erfüllt:
- sie ist zusammen mit einem Verbraucher, insbesondere mit der Steuereinheit (17) und/oder einer Sendeeinheit (9) und/oder einer Anzeigeeinrichtung, Teil einer Baueinheit (12);
- sie umfasst wenigstens eine Schwingspule;
- sie umfasst einen Permanentmagneten (15), insbesondere bestehend aus Neodym;
- sie ist derart an der Vorrichtung (1) zur Bodenverdichtung angeordnet, dass sie entweder in Bezug auf eine Bodenkontaktfläche zentrisch oder in einem Randbereich liegt.

8. Vorrichtung (1) zur Bodenverdichtung gemäß einem der vorhergehenden Ansprüche,
**dadurch gekennzeichnet,**
**dass** die Umwandlungseinrichtung (7) wenigstens einen Lineargenerator (16) umfasst.

9. Vorrichtung (1) zur Bodenverdichtung gemäß einem der vorhergehenden Ansprüche,
dadurch **gekennzeich net,**
dass die Umwandlungseinrichtung (7) einen Verbraucher, insbesondere die Steuereinheit (17) und/oder eine Sendeeinheit (9) mit elektrischer Energie versorgt.

10. Vorrichtung (1) zur Bodenverdichtung gemäß einem der vorhergehenden Ansprüche,
**dadurch gekennzeichnet,**
**dass** die Vorrichtung (1) zur Bodenverdichtung ein Anbauverdichter, eine Rüttelplatte oder eine Vibrationswalze ist.

11. Verfahren (19) zum Überwachen von Veränderungen der mit einer Vorrichtung (1) zur Bodenverdichtung erzeugten Verdichtung eines Bodens, insbesondere einer Vorrichtung (1) zur Bodenverdichtung gemäß einem der vorhergehenden Ansprüche, umfassend die Schritte:
a) Verdichten (20) eines Bodens mithilfe einer über einen Schwingungserreger (4) bei einer festgelegten Vibrationsfrequenz oder innerhalb eines Vibrationsfrequenzbereiches in Schwingungen versetzen Bodenverdichtungseinrichtung;
b) Erzeugen (21) von elektrischer Energie aus den Schwingungen der Vorrichtung mithilfe einer Umwandlungseinrichtung (7) zur Umwandlung von Vibrationen in elektrische Energie, wobei die Umwandlungseinrichtung (7) wenigstens zwei Feder-Masse-System (10, 11, 8) aufweist, die zueinander verschiedene Resonanzfrequenzen aufweisen;
c) Ermitteln und Überwachen (23) der erzeugten elektrischen Energie oder eines damit korrespondierenden Parameters beider Feder-Masse-Systeme (10, 11, 8); und
d) Korrelieren (24) der ermittelten erzeugten elektrischen Energie oder eines damit korrespondierenden Parameters mit einer Veränderung der Verdichtung des Bodens.

## Claims

1. A device for ground compaction (1), comprising
a) a frame (2) and a drive motor (3) supported by the frame (2),
b) a vibration exciter (4) driven by the drive motor (3), and
c) a ground compaction apparatus, in particular a base plate (5) or a roller drum (6), which is connected to the vibration exciter (4),
wherein the vibration exciter (4) causes the ground compaction apparatus to vibrate at a fixed vibration frequency or within a vibration frequency range during a compaction operation, and wherein a conversion device (7) for the conversion of vibrations into electric energy is provided **characterized in** which converts vibrations of the device for ground compaction (1) into electric energy,
**characterized in**
**that** the conversion device (7) comprises at least two spring-mass systems (10, 11) with different resonance frequencies, and in that a control unit (17) is provided which ascertains the degree of compaction of the ground from the electric energy obtained by the individual spring-mass systems (10, 11), in particular the respective voltages.

2. The device for ground compaction (1) according to claim 1,
**characterized in**
**that** the ratio of the resonance frequencies of the at least two spring-mass systems (10, 11) is from 1:1.5 to 1:3, preferably from 1:1.5 to 1:2.5, particularly preferably 1:1.75 to 1:2.25, and especially approximately 1:2.

3. The device for ground compaction (1) according to one of the preceding claims,
**characterized in**
**that** the two spring-mass systems (10, 11) are configured in such a way that the resonance frequency of one spring-mass system (10) is above the set vibration frequency or above the mean value of the vibration frequency range and/or the resonance frequency of the other spring-mass system (11) is below the vibration frequency or below the mean value of the vibration range.

4. The device for ground compaction (1) according to one of the preceding claims,
**characterized in**
**that** the two spring-mass systems (10, 11) are configured in such a way that the resonance frequency of one spring-mass system (10) is twice the vibration frequency or twice the mean value of the vibration frequency range and/or the resonance frequency of the other spring-mass system (11) is half the vibration frequency or half the mean value of the vibration frequency range.

5. The device for ground compaction (1) according to one of the preceding claims,
**characterized in**
**that** it comprises three spring-mass systems (10, 11, 8) which are configured in such a way that the resonance frequency f₁ of the first spring-mass system (10) corresponds to the vibration frequency or the mean value of the vibration frequency range, the resonance frequency f₂ of the second spring-mass system (11) is twice the vibration frequency or twice the mean value of the vibration frequency range, and the resonance frequency f₀ of the third spring-mass system (8) is half the vibration frequency or half the mean value of the vibration frequency range.

6. The device for ground compaction (1) according to one of the preceding claims,
**characterized in**
**that** the conversion device (7) is arranged on a ground-compacting component in direct contact with the ground, in particular on a base plate (5) or on a roller drum (6) of the ground compaction apparatus.

7. The device for ground compaction (1) according to one of the preceding claims,
**characterized in**
**that** the conversion device (7) comprises at least one of the following features:
- it is part of an assembly unit (12) together with a consumer, in particular the control unit (17) and/or a transmitting unit (9) and/or a display device;
- it comprises at least one voice coil;
- it comprises a permanent magnet (15), consisting in particular of neodymium;
- it is arranged on the device for ground compaction (1) in such a manner that it is positioned either centrally or in an edge region in relation to a ground contact surface.

8. The device for ground compaction (1) according to one of the preceding claims,
**characterized in**
**that** the conversion device (7) comprises at least one linear generator (16).

9. The device for ground compaction (1) according to one of the preceding claims,
**characterized in**
**that** the conversion device (7) supplies a consumer, in particular the control unit (17) and/or a transmitting unit (9), with electric energy.

10. The device for ground compaction (1) according to one of the preceding claims,
**characterized in**
**that** the device for ground compaction (1) is an attachable compactor, a vibratory plate compactor or a vibrating roller.

11. A method (19) for monitoring changes in the ground compaction produced with a device for ground compaction (1), in particular a device for ground compaction (1) according to one of the preceding claims, comprising the steps:
a) compacting (20) a ground with the aid of a ground compaction apparatus vibrating at a fixed vibration frequency or within a vibration frequency range by means a vibration exciter (4);
b) generating (21) electric energy from the vibrations of the device with the aid of a conversion device (7) for the conversion of vibrations into electric energy, said conversion device (7) comprising at least two spring-mass systems (10, 11, 8) with different resonance frequencies;
c) determining and monitoring (23) the electric energy generated by the two spring-mass systems (10, 11, 8) or a corresponding parameter; and
d) correlating (24) the determined electric energy generated or a corresponding parameter with a change in the compaction of the ground.

## Revendications

1. Dispositif (1) de compactage du sol, comprenant
a) un châssis (2) et un moteur d'entraînement (3) porté par le châssis (2),
b) un excitateur de vibrations (4) entraîné par le moteur d'entraînement (3), et
c) un équipement de compactage du sol, en particulier une plaque de base (5) ou un cylindre (6) de rouleau, équipement qui est relié à l'excitateur de vibrations (4),
dans lequel l'excitateur de vibrations (4) provoque la vibration de l'équipement de compactage du sol avec une fréquence de vibration fixe ou dans une gamme de fréquences de vibration pendant une opération de compactage, et dans lequel un dispositif de conversion (7) pour la conversion des vibrations en énergie électrique est prévu, qui convertit les vibrations du dispositif (1) pour le compactage du sol en énergie électrique,
***caractérisé en ce que***
le dispositif de conversion (7) comprend au moins deux systèmes masse-ressort (10, 11) ayant des fréquences de résonance différentes, et
***en ce que*** une unité de commande (17) est prévue, qui détermine le degré de compactage du sol à partir de l'énergie électrique obtenue par les systèmes masse-ressort individuels (10, 11), en particulier les tensions respectives.

2. Dispositif (1) de compactage du sol selon la revendication 1,
***caractérisé en ce que***
le rapport des fréquences de résonance desdits au moins deux systèmes masse-ressort (10, 11) est compris entre 1/1,5 et 1/3, de manière préférée entre 1/1,5 et 1/2,5, de manière particulièrement préférée entre 1/1,75 et 1/2,25, et est tout particulièrement d'environ 1/2.

3. Dispositif (1) de compactage du sol selon l'une quelconque des revendications précédentes,
***caractérisé en ce que***
les deux systèmes masse-ressort (10, 11) sont configurés de façon telle que la fréquence de résonance de l'un des systèmes masse-ressort (10) soit supérieure à la fréquence de vibration assignée ou supérieure à la valeur moyenne de la gamme de fréquences de vibration et/ou la fréquence de résonance de l'autre système masse-ressort (11) soit inférieure à la fréquence de vibration ou inférieure à la valeur moyenne de la gamme de fréquences.

4. Dispositif (1) de compactage du sol selon l'une quelconque des revendications précédentes,
***caractérisé en ce que***
les deux systèmes masse-ressort (10, 11) sont configurés de façon telle que la fréquence de résonance de l'un des systèmes masse-ressort (10) soit égale à deux fois la fréquence de vibration ou deux fois la valeur moyenne de la gamme de fréquences de vibration et/ou la fréquence de résonance de l'autre système masse-ressort (11) est la moitié de la fréquence de vibration ou la moitié de la valeur moyenne de la gamme de fréquences de vibration.

5. Dispositif (1) de compactage du sol selon l'une quelconque des revendications précédentes,
***caractérisé en ce que***
il comprend trois systèmes masse-ressort (10, 11, 8) qui sont configurés de telle façon que la fréquence de résonance f₁ du premier système masse-ressort (10) corresponde à la fréquence de vibration ou à la valeur moyenne de la gamme de fréquences de vibration, la fréquence de résonance f₂ du deuxième système masse-ressort (11) soit égale à deux fois la fréquence de vibration ou deux fois la valeur moyenne de la gamme de fréquences de vibration, et la fréquence de résonance f₀ du troisième système masse-ressort (8) soit égale à la moitié de la fréquence de vibration ou à la moitié de la valeur moyenne de la gamme de fréquences de vibration.

6. Dispositif (1) de compactage du sol selon l'une quelconque des revendications précédentes,
***caractérisé en ce que***
le dispositif de conversion (7) est installé sur un composant de compactage du sol en contact direct avec le sol, en particulier sur une plaque de base (5) ou sur un cylindre (6) de rouleau de l'équipement de compactage du sol.

7. Dispositif (1) de compactage du sol selon l'une quelconque des revendications précédentes,
***caractérisé en ce que***
le dispositif de conversion (7) comprend au moins l'une des caractéristiques suivantes :
- il constitue une partie d'une unité modulaire (12) avec un élément consommateur, en particulier l'unité de commande (17) et/ou une unité de transmission (9) et/ou un dispositif d'affichage ;
- il comprend au moins une bobine acoustique ;
- il comprend un aimant permanent (15), constitué en particulier de néodyme ;
- il est installé sur le dispositif (1) de compactage du sol d'une manière telle qu'il soit positionné soit centralement, soit dans une zone de bordure par rapport à une surface de contact avec le sol.

8. Dispositif (1) de compactage du sol selon l'une quelconque des revendications précédentes,
***caractérisé en ce que***
le dispositif de conversion (7) comprend au moins un générateur linéaire (16).

9. Dispositif (1) de compactage du sol selon l'une quelconque des revendications précédentes,
***caractérisé en ce que***
le dispositif de conversion (7) fournit de l'énergie électrique à un élément consommateur, en particulier à l'unité de commande (17) et/ou à une unité de transmission (9).

10. Dispositif (1) de compactage du sol selon l'une quelconque des revendications précédentes,
***caractérisé en ce que***
le dispositif (1) de compactage du sol est un compacteur adaptable, un compacteur à plaque vibrante, ou un rouleau vibrant.

11. Procédé (19) pour la surveillance de changements dans le compactage d'un sol effectué par un dispositif (1) de compactage du sol, en particulier un dispositif (1) de compactage du sol selon l'une quelconque des revendications précédentes, comprenant les étapes de :
a) compactage (20) d'un sol avec l'aide d'un dispositif de compactage du sol vibrant avec une fréquence de vibration fixe ou dans une gamme de fréquences de vibration au moyen d'un excitateur de vibrations (4) ;
b) génération (21) d'énergie électrique à partir des vibrations du dispositif avec l'aide d'un dispositif de conversion (7) pour la conversion des vibrations en énergie électrique, ledit dispositif de conversion (7) comprenant au moins deux systèmes masse-ressort (10, 11, 8) avec des fréquences de résonance différentes ;
c) détermination et surveillance (23) de l'énergie électrique générée par les deux systèmes masse-ressort (10, 11, 8) ou d'un paramètre correspondant ; et
d) corrélation (24) de l'énergie électrique générée déterminée ou d'un paramètre correspondant avec un changement dans le compactage du sol.
